# EUROPÄISCHE PATENTANMELDUNG

(11) **EP 2 090 282 A1**
(43) Veröffentlichungstag der Anmeldung: **19.08.2009**
(21) Anmeldenummer: 09150644.4
(22) Anmeldetag: 15.01.2009
(51) Int. Cl.: A61K 8/06, A61K 8/67, A61Q 3/00

(54) **Kosmetische oder medizinische Zubereitung enthaltend Biotin**

(30) Priorität: 12.02.2008 DE 102008008554; 03.12.2008 DE 202008016055 U
(71) Anmelder: Sebapharma GmbH & Co., 56136 Boppard-Bad Salzig (DE)
(72) Erfinder: Gottfreund, Joachim, 56154, Boppard (DE); Meyer, Thomas, 56281, Emmelshausen (DE)
(74) Vertreter: Lippert, Stachow & Partner

(57) **Zusammenfassung**

Die Erfindung betrifft eine kosmetische oder dermatologische Zubereitung enthaltend eine optisch transparente Biotin und/oder ein Biotinderivat enthaltende Mikroemulsion, wobei die Mikroemulsion oder die Zubereitung insgesamt enthält:
a) 0,025 bis 5 Gew.-% Biotin und/oder mindestens ein physiologisch verträgliches Biotinderivat,
b) 2 bis 25 Gew.-% einer Ölkomponente,
c) 10 bis 60 Gew.-% eines oder mehrerer Cotenside, und
d) 10 bis 75 Gew.-% Wasser.

Die erfindungsgemäße Zubereitung hat sich zur Behandlung von von Nagelfunktionsstörungen oder Nagelerkrankungen, insbesondere von Nagelspliss, als besonders wirksam herausgestellt.

## Beschreibung

Die Erfindung betrifft eine kosmetische oder dermatologische, einschließlich medizinische, Zubereitung enthaltend eine optisch transparente Mikroemulsion, die Biotin und/oder ein Biotinderivat enthält.

Spröde und brüchige oder splitternde Finger- oder Fußnägel stellen ein weit verbreitetes Problem dar, welches oftmals mit einem großen Leidensdruck der betroffenen Personen verbunden ist.

Der therapeutische Nutzen von Biotin (Vitamin H) wurde oftmals unterschiedlich beschrieben. Es sind teilweise positive Studien bekannt, oftmals sind die durch Verabreichung von Biotin erzielten Ergebnisse jedoch enttäuschend (siehe z.B. Worret, Gehring; Kosmetische Dermatologie, Springer-Verlag, Seite 255-257). Hierbei ist oftmals eine orale Verabreichung notwendig (siehe z.B. Raab, Kindl; Pflegekosmetik; Wissenschaftliche Verlagsgesellschaft mbH Stuttgart, 4. Auflage, Seite 260). Auch nach Mutschler, Arzneimittelwirkungen, Wissenschaftliche Verlagsgesellschaft mbH Stuttgart, 8. Auflage, Seite 751, ist die Wirksamkeit von Biotin bei erhöhter Brüchigkeit von Nägeln und Haaren fraglich.

Die Ursachen für die einander teilweise widersprechenden Ergebnisse bezüglich der Wirksamkeit von Biotin insbesondere bei der Behandlung spröder, brüchiger und splitternder Nägel sind nur schwer erklärbar, möglicherweise hängen diese auch damit zusammen, dass bei der Verabreichung von Biotin, insbesondere bei oraler Verabreichung, nur kleine Mengen von Biotin tatsächlich zur Nagelwurzel gelangen. Ausgehend von einem täglichen Biotin-Bedarf von 0,1 bis 0,2 mg ist es zur Erzielung eines positiven Effektes auf den Nagel notwendig, dem Körper oral eine bis zu 50-fache Menge an Biotin zuzuführen, was eine beträchtliche Überdosierung darstellt.

Weiterhin ist eine topische Applikation von Biotin auf die Haut oder den Nagel bekannt, hierbei ergeben sich jedoch beträchtliche Schwierigkeiten in Bezug auf die Penetration des Biotins durch die Haut oder den Nagel. Sowohl die Hornhaut (stratum corneum) als auch Keratin stellen eine erhebliche Diffusionsbarriere für Biotin dar, die durch eine herkömmliche Galenik wie z.B. eine W/O- oder O/W-Emulsion, nicht wirksam überwunden werden kann.

Zur Verbesserung der Diffusion von Biotin in die Haut ist die Verwendung von Penetrationsenhancern wie Dimethylsulfoxid, Dimethylacetamid, Dimethylformamid oder Azon (z.B. US 5,166, 168) oder auch Harnstoff vorgeschlagen worden. Die Verwendung derartiger Substanzen ist jedoch zum Teil aus toxikologischer und allergologischer Sicht umstritten und problematisch. Weiterhin ist der Nachweis einer ausreichenden Penetration von Biotin durch den Nagel auch unter Verwendung von Penetrationsenhancern bisher nicht zuverlässig nachgewiesen.

In Bezug auf die Applikation auf Hand- und/oder Fußnägel ist außer der Anwendung von O/W- bzw. W/O-Emulsionen, die sich als nicht ausreichend wirksam erwiesen haben, die Anwendung als Lack als bevorzugt beschrieben, beispielsweise in der EP 679 383 B1. Derartige Nagellacke oder Pflege-Nagelgrundierungen auf Basis organischer Lösungen sind jedoch ebenfalls hochproblematisch, da Lacke einen hohen Gehalt an organischen Lösungsmitteln wie Toluol, Xylol, Ester niederer Fettsäuren wie Butylacetat, Ethylacetat oder niedere Alkohole wie Isopropanol aufweisen, was jedoch zu beträchtlichen Hautirritationen, Austrocknung der Haut und dergleichen führen kann.

Andererseits wird ein medizinisch günstiger Zustand des Nagels nicht nur durch die fehlende Sprödigkeit und Brüchigkeit beschrieben, ein gesunder Nagel soll auch ein ausreichend hohes Quellvermögen haben.

Eine kosmetische oder dermatologisch unbedenkliche Zubereitung, durch welche Nagelspliss in vergleichsweise kurzer Zeit wirksam vermindert und zudem der Quellfaktor der Nägel erhöht werden kann, liegt somit noch nicht vor.

Der Erfindung liegt somit die Aufgabe zugrunde, eine kosmetische oder dermatologische Zubereitung bereitzustellen, welche stabil und zur Behandlung von Nagelspliss hochwirksam ist sowie den Quellfaktor der Nägel erhöht, so dass in relativ kurzer Zeit ein positiver Erfolg sichtbar ist.

Die Aufgabe wird durch eine kosmetische oder dermatologische, einschließlich medizinische, Zubereitung nach Anspruch 1 gelöst, welche eine optisch transparente, Biotin und/oder mindestens ein Biotinderivat enthaltende Mikroemulsion enthält, wobei die Zubereitung oder die Mikroemulsion 0,05 - 2 Gew.-% Biotin und/oder mindestens ein Biotinderivat, 2 - 25 Gew.-% einer Ölkomponente, 10 - 60 Gew.-% eines oder mehrere Cotenside und 10 - 75 Gew.-% Wasser enthält.

Überraschenderweise hat sich die erfindungsgemäße Zubereitung trotz eines vergleichsweise niedrigen Gehaltes an Biotin und/oder Biotinderivat als hochwirksam erwiesen, so dass diese zuverlässig die Diffusionsbarriere des Nagels durchdringen kann, aber auch andere Biotin-Diffusionsbarrieren wie beispielsweise die Hornhaut (stratum corneum), welche im Allgemeinen aufgrund deren Lipophilie und geringen Hydratation im Normalzustand für Biotin ein besonders hohe Penetrationsbarriere darstellen. Weiterhin kann die erfindungsgemäße Zubereitung oder die Mikroemulsion praktisch frei von Penetrationsenhancern wie z.B. Harnstoff, Dimethylsulfoxid und dergleichen sein oder diese nur in geringem Gehalt aufweisen.

Von besonderer Bedeutung ist hierbei, dass aus diesen erfindungsgemäßen Zubereitungen der Wirkstoff Biotin wesentlich besser und tiefer durch Nägel aber auch durch Haut penetriert. Nach der Penetration durch den Nagel ist Biotin an der Keratinozytenreifung beteiligt, was optisch an der Verbesserung der Nagelqualität sichtbar wird. Es ist deshalb auch möglich, den Gehalt an Wirkstoffen in diesen Zubereitungen sehr viel niedriger zu halten, als in herkömmlichen Zubereitungen.

Durch die erfindungsgemäße Zubereitung kann innerhalb von zwei bis drei Monaten Nagelspliss sehr deutlich reduziert und der Quellfaktor von Nägeln (Hand- und/oder Fußnägeln) erhöht werden. Dies gilt für mammale, insbesondere für humane Nägel.

Ferner wird durch die Zubereitung das optische Erscheinungsbild der Nägel in appliziertem Zustand nicht negativ beeinflusst.

Biotin (3aS,4S,6aR)-2-Oxohexahydrothieno[3,4-d]-imidazol-4-valeriansäure) und/oder die Biotinderivate liegen vorzugsweise in der nachfolgend dargestellten stereoisomeren Form vor.

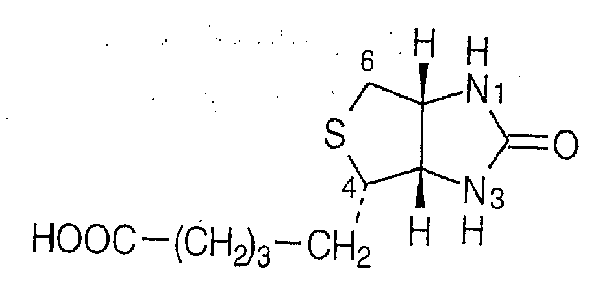

Gegebenenfalls können jedoch auch andere stereoisomere Formen des Biotin und/oder eines oder mehrere der Biotinderivate, optische Isomere, Diostereomere oder Racemate derselben eingesetzt werden.

Als Biotinderivate kommen insbesondere solche Verbindungen in Frage, die unter physiologischen Bedingungen Biotin freisetzen oder den Biotingehalt an der Nagelwurzel erhöhen.

Als Biotinderivate kann gegebenenfalls ein Carboxybiotin eingesetzt werden.

Allgemein kann am N1 und/oder am N3 Atom des Biotins oder Biotinderivates das H-Atom unabhängig voneinander ersetzt sein durch eine Gruppe ausgewählt aus -COOH Gruppe (Carboxybiotin), Carboxyalkylgruppe (-COOR¹), eine -CONHR¹-Gruppe, eine -CONR¹R²-Gruppe ersetzt sein, wobei R¹ und R² gleich oder verschieden sein können. Insbesondere können beide N1- und N3-H-Atome durch jeweils die gleiche Gruppe ersetzt sein. Es kann auch eines der H-Atome am N1 oder N3 durch eine Carboxy- oder Carboxaalkylgruppe und das andere H-Atom durch eine Amidgruppe unter Bildung einer gemischten Ester-Amid-Verbindung ersetzt sein.

Zusätzlich oder unabhängig hiervon kann die Carboxygruppe des - (CH₂)₄COOH Restes unter Ausbildung einer -(CH₂)₄COOR¹-Gruppe verestert oder zu einem primären oder sekundären Amid - (CH₂)₄CONHR¹ oder -(CH₂)₄CONR¹R² funktionalisiert sein, wobei die N1- und N3-H-Atome nicht substituiert oder wie oben angegeben substituiert sein können. Im Falle von Carboxybiotin können Mono-, Di- oder Triester vorliegen.

R¹ und R² können unabhängig voneinander ausgewählt sein aus der Gruppe C₁- bis C₂₀-Alkylrest, insbesondere C₁- bis C₁₂-, C₁- bis C₄- oder bis C₆- oder C₂- bis C₈-Alkylrest. Der Alkylrest kann linear, verzweigt oder zyklisch sein. Insbesondere können R¹ und R² unabhängig voneinander Metyhl, Ethyl, Propyl oder Butyl sein .Ist der R¹-Rest Teil einer Estergruppe, so kann der entsprechende Alkohol auch Teil einer Hydroxycarbonsäure oder Hydroxyaminosäure sein, vorzugsweise mit 1 bis 20, insbesondere 2 bis 12, 2 bis 8 oder 4 bis 12 Kohlenstoffatomen.

Zugleich oder unabhängig hiervon kann das Amid von Biotin (unter Amidierung der (CH₂)₄COOH-Gruppe) und/oder Carboxybiotin (am N1 und/oder N3-Atom carboxyliert) auch jeweils unter Bildung eines primären oder sekundären Amins (vorzugsweise jeweils primäres Amin) mit einer Aminosäure, Dipeptid, Tripeptid, Polypeptid oder Enzym amidiert sein. Das Amin ist vorzugsweise physiologisch verträglich. Insbesondere kann nur die (CH₂)₄COOH-Gruppe amidiert sein oder die (CH₂)₄COOH-Gruppe und die Nl-arboxy-Gruppe).

Gegebenenfalls können bei jeder der oben genannten Verbindungen, einschließlich Biotin, eines oder beide der 3a-H und/oder 6a-H Atome unabhängig voneinander durch eine C₁- bis C₄-AlkylGruppe substituiert sein, insbesondere unabhängig voneinander Methyl oder Ethyl.

Erfindungsgemäß eingesetzt werden können jeweils unabhängig voneinander auch die physiologisch verträglichen Salze von Biotin und/oder Biotinderivaten, insbesondere den oben genannten Derivaten, wie beispielsweise Alkalisalze (insbesondere Li, Na, K-Salze), Erdalkalisalze (insbesondere Mg und Ca-Salze), Ammoniumsalze oder Salze von anderen physiologisch verträglichen organischen Basen. Die Salze des Carboxybiotins und dessen Derivaten können jeweils ein-, zwei oder dreiwertige Salze sein.

Der Gehalt an Biotin und/oder Biotinderivat in der Zubereitung oder der Mikroemulsion kann in Summe ≥ 0,01 Gew.-% oder ≥ 0,025 oder ≥ 0,05 Gew.-% betragen, vorzugsweise zwischen 0,1 und 1 Gew.-% oder im Bereich von 0,1 - 0,5 Gew.-% liegen, besonders bevorzugt im Bereich von 0,15 - 0,3 Gew.-%. Gehalte von ≥ 0,1 - 0,15 Gew.-% oder ≥ 0,18 - 0,2 oder ≥ 0,22 - 0,25 Gew.-% Biotin haben sich als besonders bevorzugt erwiesen, um auch den Quellfaktor der behandelten Nägel zu erhöhen, wobei zugleich eine gegenüber niedrigeren Biotingehalten nochmals gesteigerte Nagelqualität festgestellt wird. Gegebenenfalls kann der Biotingehalt in der Zubereitung oder Mikroemulsion auch ≥ 0, 5 - 1 Gew.-% oder ≥ 1,5 - 2 Gew.-% betragen, beispielsweise bis zu 2,5 - 3 Gew.-% oder bis zu 4 - 5 Gew.-% betragen oder höher, auch wenn dies nicht erforderlich ist.

Die erfindungsgemäßen kosmetischen Zubereitungen stellen eine Sonderform von Mikroemulsionen dar, die sich in ihren Eigenschaften von herkömmlichen Emulsionstypen, nämlich Öl in Wasser, Wasser in Öl oder Triple-Emulsionen unterscheidet. Es handelt sich vielmehr um eine Art Mikro-Emulsion, die ein Gleichgewicht zwischen micellaren Strukturen und lamellaren einphasigen Strukturen aufweist. Die Teilchengröße der Emulsionsteilchen kann überwiegend (in Vol.-% oder Gew.-%) im Bereich zwischen 5 und 50 nm liegen, vorzugsweise im Bereich von 10 - 30 nm oder insbesondere im Bereich von 10 - 20 nm. Diese Zubereitungen sind daher optisch transparent und isotop, wodurch sie sich optisch von herkömmlichen Cremes und Lotionen unterscheiden. Erstaunlicherweise sind diese Zubereitungen thermodynamisch stabil, zeigen keine Phasentrennung und sind somit praktisch unbegrenzt lagerfähig, während herkömmliche W/O- bzw. O/W-Emulsionen diese guten Eigenschaften nicht aufweisen. Von besonderer Bedeutung ist, dass aus diesen erfindungsgemäßen Zubereitungen die Wirkstoffe Biotin und/oder Biotinderivate wesentlich besser und tiefer in den Nagel oder gegebenenfalls auch die Haut eindringen und dort die Wirkung entfalten können, ohne dass es zu Hautreizungen kommt.

In der erfindungsgemäßen Zubereitung oder Mikroemulsion kann die Ölkomponente in einem Gehalt von 2 - 20 Gew.-%, vorzugsweise 4 - 15 Ges.-%, besonders bevorzugt im Bereich von 5 - 10 Gew.-% vorliegen.

Als Ölkomponenten können eingesetzt werden: Mineralöl; natürliche Öle und Ester von linearen C₆-C₃₀-Fettsäuren (vorzugsweise C₈-C₂₆-Fettsäuren oder C₈-C₂₂-Fettsäuren) mit linearen C₂-C₃₀-Fettalkoholen (vorzugsweise C₈-C₂₆- oder C₈-C₁₈- Fettalkoholen); Ester von verzweigten C₄-C₂₂-Carbonsäuren (vorzugsweise C₆-C₁₆ oder C₈-C₁₃-Carbonsäuren) mit linearen C₄-C₃₀-Fettalkoholen (vorzugsweise C₈-C₂₆- oder C₈-C₂₂-Fettalkoholen); bevorzugt Ester von linearen C₄-C₃₀-Fettsäuren (vorzugsweise C₈-C₂₆- oder besonders bevorzugt C₈-C₂₂-Fettsäuren) mit verzweigten Alkoholen (insbesondere Alkylalkoholen mit vorzugsweise 4 bis 20, 6 bis 16 oder 6 bis 12 Kohlenstoffatomen wie 2-Ethylhexanol); Ester von linearen und/oder verzweigten Fettsäuren (vorzugsweise C₄-C₃₀-Fettsäuren, C₆-C₂₂- oder besonders bevorzugt C₈-C₁₈-Fettsäuren) mit mehrwertigen Alkoholen; wobei vorzugsweise bei jedem der oben genannten Ester die Fettsäure und/oder der Alkohol ≥ 8 C-Atome aufweist; Triglyceride auf Basis C₄-C₂₈-oder C₄-C₁₈-Fettsäuren (vorzugsweise C₆-C₁₀-Fettsäuren); flüssige Mono-/Di-/Triglycerid-Mischungen auf Basis von C₄-C₂₈-Fettsäuren (vorzugsweise C₆-C₁₈- oder C₆-C₁₀-Fettsäuren).

Besonders bevorzugt sind Ethylcocoat, Hexyldecyllaurate und Ethylhexylstearate.

Der Gehalt an Cotensid der erfindungsgemäßen Zubereitung oder der Mikroemulsion kann im Bereich von 20 - 60 Gew.-%, vorzugsweise im Bereich von 25 - 55 Gew.-% oder im Bereich von 30 - 50 Gew.-% liegen, gegebenenfalls auch im Bereich von 35 - 45 Gew.-%. Unter Cotensid im Sinne der Erfindung seien hierbei oberflächenaktive Stoffe verstanden, die selber jedoch keine Micellenbildung bewirken.

Als Cotenside können einzeln oder als Gemisch eingesetzt werden:
Mono-, Di-, Triglyceride von C₄-C₁₈ linearen oder gegebenenfalls auch verzweigten Fettsäuren (vorzugsweise C₆-C₁₂ oder C₆-C₈ Fettsäuren) mit 4 bis 20 Mol Ethylenoxid (vorzugsweise 6 bis 10 Mol); Mono-, Di- und Triester von C₆-C₃₀ linearen oder gegebenenfalls auch verzweigten Fettsäuren (vorzugsweise C₁₅-C₂₀ Fettsäuren) mit Glycerinpolymeren, die zwischen 2 und 30 (vorzugsweise zwischen 4 und 20 oder 4 und 10) Glycerineinheiten enthalten; Mono-, Di- und Triester von C₆-C₂₆ linearen oder gegebenenfalls auch verzweigten Fettsäuren (vorzugsweise C₈-C₂₀- oder C₁₀-C₁₅- Fettsäuren) mit Sorbitol, die zwischen 5 und 120 EO-Einheiten (vorzugsweise zwischen 10 und 80 oder zwischen 15 und 60 EO-Einheiten) enthalten können.

Ferner sind geeignet Sorbitanester wie Sorbitanmonoisostearat, Sorbitansesquiisostearat, Sorbitandiisostearat, Sorbitantriiosstearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmono-ricinoleat, Sorbitansesquiricinoleat, Sorbitandiricinoleat, Sorbitantriricinoleat, Sorbitanmonohydroxystearat, Sorbitansesquihydroxystearat, Sorbitandihydroxy-stearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 300, vorzugsweise 5 bis 80 oder 20 bis 60 Mol Ethylenoxid an die genannten Sorbitanester.

Besonders geeignet sind Gemische aus Polysorbat 20 mit Polyglyceryl-6 Dioleate oder PEG-8 Caprylic-capric Glyceride.

Neben dem Cotensid können weiterhin ein oder mehrere Tenside in der Mikroemulsion enthalten sein, beispielsweise anionische, kationische und/oder neutrale Tenside. Unter Tensiden im Sinne der Erfindung seien hierbei oberflächenaktive Stoffe verstanden, die in geeigneten Konzentrationen eine Micellenbildung bewirken. Die Tenside können Alkylsulfatsalze darstellen, beispielsweise mit C12 bis C30-Ketten, insbesondere C12 bis C24-Ketten, das Gegenion kann ein Alkalimetallion, Ammoniumion, eine protonierte organische Base oder dergleichen sein. Das anionische Tensid kann beispielsweise Natriumdodecylsulfat sein. Gegebenenfalls können auch kationische Tenside wie quarternäre Ammoniumsalze eingesetzt werden, wie beispielsweise Hexadecyltrimethylammoniumbromid. Weiterhin können auch nichtionische Tenside eingesetzt werden, wie z.B. langkettige Alkylpolyethylenoxidether, die beispielsweise 10 - 80, 20 - 60 oder 30 - 60 Ethylenoxideinheiten aufweisen können, insbesondere auch ca. 50 Einheiten. Weiterhin können gegebenenfalls biokompatible Tenside wie Lecithin oder Cholsäure vorhanden sein.

Tenside können in einem Gehalt von 10 - 50 Gew.-%, bzw. 15 - 45 Gew.-% oder 20 - 40 Gew.-% in der Zubereitung oder der Mikro-emulsion vorhanden sein. Gegebenenfalls können die Tenside auch in einem Gehalt von ≤ 20 - 30 Gew.-%, insbesondere ≤ 10 - 15 Gew.-% oder ≤ 5 - 8 Gew.-% vorhanden sein. Der Tensidgehalt der Mikroemulsion kann gegebenenfalls auch ≤ 3 - 5 Gew.-% oder ≤ 1 - 2 Gew.-% betragen oder die Mikroemulsion kann im Wesentlichen frei von Tensiden sein.

Der pH-Wert der Mikroemulsion oder der Zubereitung insgesamt kann auf einen Wert von 5,5 ± 1 oder vorzugsweise 5,5 ± 0,5 eingestellt sein. Insbesondere kann der pH-Wert durch physiologisch unbedenkliche organische Säuren wie Carbonsäuren, insbesondere Ketocarbonsäuren (z.B. Alphaketocarbonsäuren), Aminosäuren, Hydroxycarbonsäuren (z.B. Alphahydroxycarbonsäuren, wie Milchsäure, Zitronensäure, Apfelsäure usw.) eingestellt sein.

Weiterhin können die erfindungsgemäßen Zubereitungen Pflegestoffe und Vitamine enthalten, wie Vitamin-A-Acetat, Retinol, Vitamin-E-Acetat, Tocopherol, Panthenol, Ceramide I-VI oder Gemische derselben. Der Gesamtgehalt an Pflegestoffen und Vitaminen kann bis zu 10 - 15 Gew.-% betragen. Schließlich können die Zubereitungen 0,1 bis 2,0 Gew.-%, vorzugsweise 0,5 bis 1 Gew.-% Konservierungsmittel enthalten. Als Konservierungsstoffe kommen in Frage Phenoxyethanol, Benzylalkohol, Ethyl-, Methyl-, Butyl-, Propyl-, Isobutylparabene, die genannten Parabene einzeln oder in Kombination.

Vorzugsweise weist die Zubereitung keinen Penetrationsenhancer auf oder lediglich in geringen Gehalten von beispielsweise < 0,8 - 1 Ges.-%, ≤ 0,3 - 0,5 Gew.-% oder ≤ 0,2 - 0,1 Gew.-% der Zubereitung oder Mikroemulsion.

Vorzugsweise ist der Gehalt organischer Lösungsmittel in der Mikroemulsion oder der Zubereitung ≤ 30 - 40 Gew.-%, ≤ 20 - 25 Gew.-% oder ≤ 10 - 15 Gew.-%, besonders bevorzugt ≤ 5 - 7 Gew.-% oder ≤ 2 - 3 Gew.-% oder besonders bevorzugt ≤ 1 Gew.-%. Die erfindungsgemäße Mikroemulsion oder Zubereitung kann auch im Wesentlichen frei von organischen Lösungsmitteln wie Aromaten (z. B. Toluol, Xylol oder dergleichen), niederen Alkoholen wie z. B. einwertigen C1 bis C6-Alkoholen, insbesondere Ethanol, Propanol (einschließlich Isopropanol), Butanol oder dergleichen sein, Estern niederer Alkohole mit niederen Carbonsäuren wie beispielsweise Butyl-, Propyl-, Ethylacetat oder dergleichen, gegebenenfalls auch allgemein Ester von C1- bis C6-Alkoholen mit C1- bis C6-Carbonsäuren.

Die erfindungsgemäße Zubereitung kann insbesondere eine topisch anwendbare galenische Formulierung sein. Die Mikroemulsion oder die Zubereitung insgesamt kann transparent oder optisch klar sein.

Die erfindungsgemäße Zubereitung ist insbesondere gegen Nagelspliss und zur Erhöhung des Quellfaktors von Nägeln, insbesondere bei Säugetieren oder bei Menschen, anwendbar. Die Zubereitung kann jedoch auch allgemein zur Behandlung von Nagelfunktionsstörungen oder Nagelerkrankungen Anwendung finden, gegebenenfalls auch zur Behandlung von Haarausfall, zur Verbesserung der Haarqualität oder als dermatologische Zubereitung, wie beispielsweise bei Alopecie, atopischer oder seborrhoischer Dermatitis, gegen Fältchenbildung oder dergleichen.

Die Erfindung wird nachfolgend anhand von Ausführungsbeispielen beschrieben, die bevorzugte Zubereitungen darstellen.

### Beispiel 1

| | Gew.-% |
|---|---|
| Polyglyceryl-6-Dioleate | 10-15 |
| Ethylcocoate | 5-10 |
| PEG-8 Caprylic/Capric Glyceride | 10-15 |
| Polysorbate 20 | 20-25 |
| Pflegestoffe | 0,5-10 |
| Biotin | 0,05-1 |
| organische Säure | 0,1-1 |
| Parfum | 0,5-5 |
| Konservierungsmittel | 0,2-1 |
| Wasser | ad. 100 |
| pH-Wert: | 5,5 ± 0,5 |

### Beispiel 2

| | Gew.-% |
|---|---|
| Polyglyceryl-6-Dioleate | 10-15 |
| Hexyldecyllaurate | 5-10 |
| Hexyldecanol | 5-10 |
| PEG-8 Caprylic/Capric Glyceride | 10-15 |
| Polysorbate 20 | 20-25 |
| Pflegestoffe | 0,5-10 |
| Biotin | 0,05-1 |
| organische Säure | 0,1-1 |
| Parfum | 0,5-5 |
| Konservierungsmittel | 0,2-1 |
| Wasser | ad. 100 |
| pH-Wert: | 5,5 ± 0,5 |

### Beispiel 3

| | Gew.-% |
|---|---|
| Polyglyceryl-6-Dioleate | 10-15 |
| Ethylhexylstearate | 10-15 |
| PEG-8 Caprylic/Capric Glyceride | 10-15 |
| Polysorbate 20 | 20-25 |
| Pflegestoffe | 0,5-10 |
| Biotin | 0,05-1 |
| organische Säure | 0,1-1 |
| Parfum | 0,5-5 |
| Konservierungsmittel | 0,2-1 |
| Wasser | ad. 100 |
| pH-Wert: | 5,5 ± 0,5 |

Die Wirksamkeit der erfindungsgemäßen Zubereitungen wurde an einer Gruppe von 20 Probanden nachgewiesen, die spröde und brüchige Fingernägel aufwiesen und bei denen das Quellverhalten von Nagelmaterial nach Inkubation mit 2,5 n NaOH unter dem Faktor 2,5 lag.

Durch die Untersuchungen konnte die Wirksamkeit der topischen Verwendung von Biotin bei reduzierter Nagelqualität nachgewiesen werden. Primärer Bewertungsparameter war hierbei die Nageldicke, die mit einer Verbesserung der Nagelqualität und einer Verminderung von Nagelspliss einherging. Zur Bestimmung der Nageldicke wurde ein Nagelspan vom distalen Nagelrand im rechten Winkel zur Nagelplatte aufgeblockt und ein 10µ dicker Kryostatschnitt angefertigt, an dem die Dicke mikroskopisch bestimmt werden konnte.

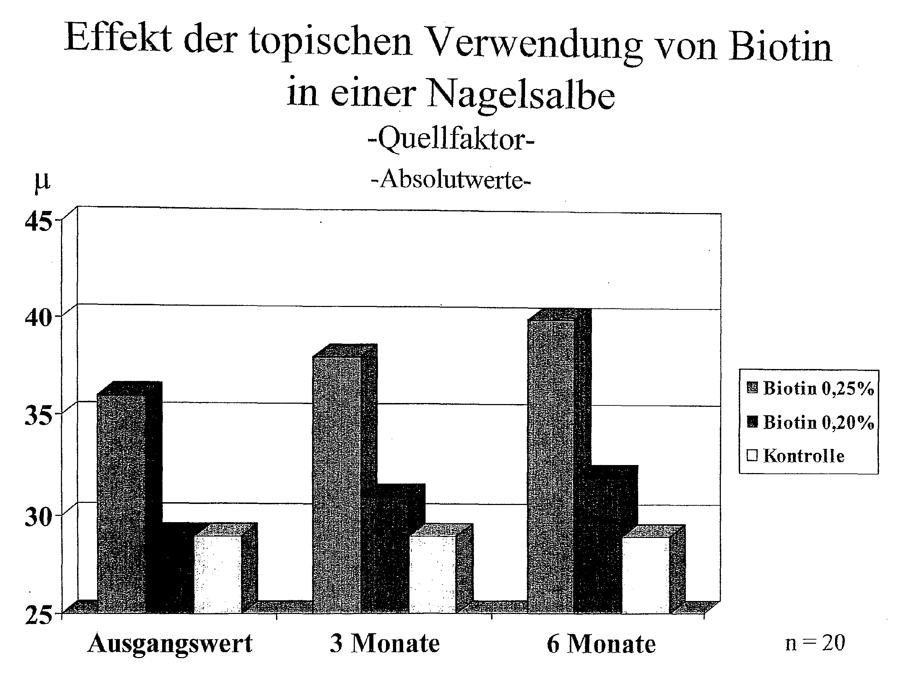

Bei der Bestimmung der Ausgangswerte wurde eine vergrößerte Nageldicke bei Anwendung der erfindungsgemäßen Zubereitung festgestellt, insbesondere bei Biotingehalten von > 0,2 Gew.-% Biotin, insbesondere ≥ 0,25 Gew.-% Biotin. Die Behandlung einer Zubereitung enthaltend 0,25 Gew.-% Biotin hatte bereits nach drei Monaten zu einer Zunahme der Nageldicke geführt, die nach 6 Monaten noch gesteigert werden konnte (siehe Abb. 1).

Weiterhin wurde das Quellverhalten von Nagelkeratin auf Natronlauge untersucht, welches als sensibler Parameter für die Nagelqualität gilt (siehe Zaun et al, die Quelleigenschaften von Nagelmaterial in Natronlauge bei der Bestimmung mit einer standardisierten Methode, Ärztl. Kosm. 1976, 6: 115-119; Zaun H. in Ärztl. Kosm. 1981, 11: 242-244). Die Inkubation und mikroskopische Vermessung des Nagelkeratins erfolgte in vitro an einem 10µ dicken Kryostatschnitt unter einem locker aufliegenden Deckgläschen. Von einer reduzierten Nagelqualität kann ausgegangen werden, wenn die durch 2,5 nNaOH provozierte Quellung kleiner als der Faktor 2,5 ist.

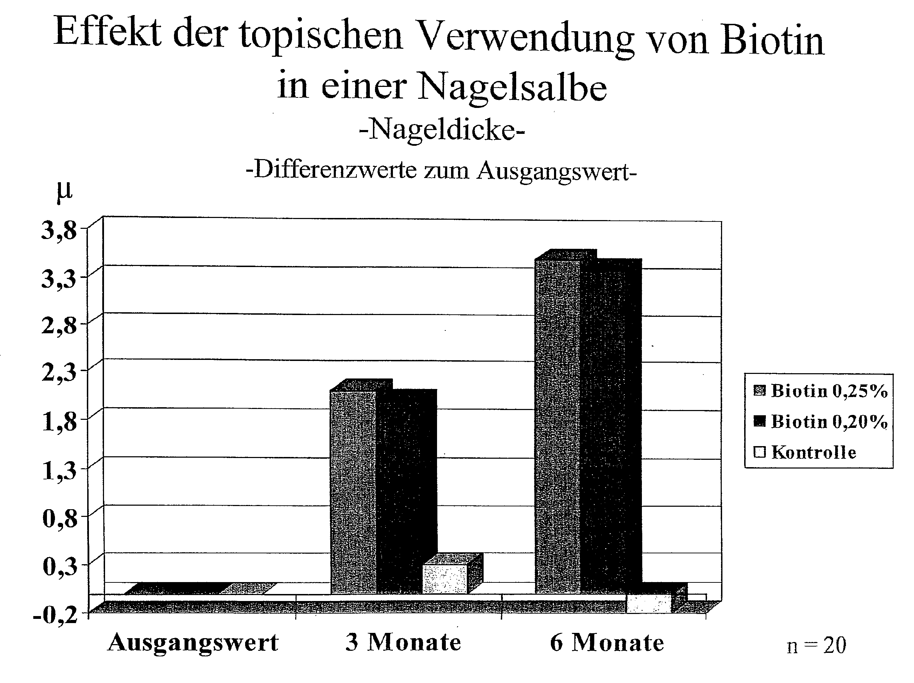

Durch Anwendung der erfindungsgemäßen Zubereitung enthaltend 0,2 Gew.-% Biotin über 4 bis 6 Monate konnte das Quellverhalten des Nagelkeratins signifikant verbessert werden (siehe Abb. 2).

Bei der topischen Anwendung der erfindungsgemäßen Zubereitungen kann somit nach ca. 2 bis 3 Monaten eine signifikante Verbesserung der Nageldicke und nach ca. 4 bis 6 Monaten eine signifikante Verbesserung des Quellverhaltens des Nagelmaterials auf NaOH festgestellt werden. Die Wirksamkeit der erfindungsgemäßen Zubereitung bei topischer Anwendung ist hierdurch belegt. Aufgrund der verschiedenen beobachteten Effekte ergibt sich ferner, dass eine direkte Biotinwirkung am Nagelbett und damit eine Penetration des Biotins durch die Nagelplatte gefunden haben. Diese Wirkungen wurden jeweils gegenüber einer Blindprobe ohne Biotin verifiziert. Die Effektivität der erfindungsgemäßen Zubereitung übertrifft hierbei sogar die einer systemischen Behandlung durch orale Verabreichung. Weiterhin wurde nachgewiesen, dass die Verwendung einer herkömmlichen W/O-Emulsion mit Biotin anstelle der erfindungsgemäßen Mikroemulsion das gleiche Ergebnis wie bei der Blindprobe ohne Biotin erhalten wurde.

Zur Erhöhung des Quellfaktors haben sich somit erfindungsgemäße Zubereitungen mit einem Gehalt von Biotin von > 0,2 Gew.-%, insbesondere ≥ 0,23 - 0,25 Gew.-% Biotin als besonders bevorzugt herausgestellt. Ein Biotingehalt von ≥ 0, 75 - 1 Gew.-% oder auch ≥ 0,5 - 0,75 Gew.-% ist hierbei nicht unbedingt erforderlich, wenn auch möglich.

## Patentansprüche

1. Kosmetische oder dermatologische Zubereitung enthaltend eine optisch transparente Biotin und/oder mindestens ein Biotinderivat enthaltende Mikroemulsion, wobei die Mikro-emulsion oder die Zubereitung insgesamt enthält:
a) 0,025 bis 5 Gew.-% Biotin und/oder mindestens ein physiologisch verträgliches Biotinderivat,
b) 2 bis 25 Gew.-% einer Ölkomponente,
c) 10 bis 60 Gew.-% eines oder mehrerer Cotenside, und
d) 10 bis 75 Gew.-% Wasser.

2. Zubereitung nach Anspruch 1, **dadurch gekennzeichnet, dass** die Mikroemulsion oder die Zubereitung insgesamt 0,05 bis 2 Gew.-% Biotin und/oder mindestens ein physiologisch verträgliches Biotinderivat enthält.

3. Zubereitung nach Anspruch 1 oder 2, **dadurch gekennzeichnet, dass** das Biotinderivate ausgewählt ist aus der Gruppe der Biotinester, Biotinamide, Carboxybiotin, Carboxybiotinester und Carboxybiotinamide, einschließlich jeweils der physiologisch verträglichen Salze derselben.

4. Zubereitung nach einem der Ansprüche 1 bis 3, **dadurch gekennzeichnet, dass** der Gehalt an Biotin und/oder dem Biotinderivat 0,1 bis 0,5 Gew.-% beträgt.

5. Zubereitung nach einem der Ansprüche 1 bis 4, **dadurch gekennzeichnet, dass** die Ölkomponente in einem Gehalt von 4 bis 15 Gew.-% vorliegt.

6. Zubereitung nach einem der Ansprüche 1 bis 5, **dadurch gekennzeichnet, dass** der Gehalt an Cotensid 30 bis 50 Gew.-% beträgt.

7. Zubereitung nach einem der Ansprüche 1 bis 6, **dadurch gekennzeichnet, dass** der Gehalt an Wasser 20 bis 60 Gew.-% beträgt.

8. Zubereitung nach einem der Ansprüche 1 bis 9, **dadurch gekennzeichnet, dass** eine physiologisch verträgliche organische Säure enthalten ist.

9. Zubereitung nach Anspruch 8, **dadurch gekennzeichnet, dass** der Gehalt an der organischen Säure 0,1 bis 2,5 Gew.-% beträgt.

10. Zubereitung nach einem der Ansprüche 1 bis 9, **dadurch gekennzeichnet, dass** der pH-Wert der Zubereitung oder der wässrigen Phase derselben auf einen pH-Wert von 5,5 ± 1,0 eingestellt ist.

11. Zubereitung nach einem der Ansprüche 1 bis 10, **dadurch gekennzeichnet, dass** die Größe der Emulsionsbestandteile der Mikroemulsion zumindest teilweise oder überwiegend in einem Bereich zwischen 5 nm und 50 nm liegen.

12. Zubereitung nach einem der Ansprüche 1 bis 11, **dadurch gekennzeichnet, dass** die Zubereitung einen oder mehrere der Bestandteile von Pflegestoffen, Vitaminen und Konservierungsmittel aufweist.

13. Zubereitung nach einem der Ansprüche 1 bis 12, **dadurch gekennzeichnet, dass** die Zubereitung zusätzlich Tocopherol, Tocopherylacetat, Retinol, Retinylpalmitat, Retinylacetat, Panthenol, Ceramide I-VI sowie Gemische derselben enthält.

14. Verwendung einer kosmetischen oder dermatologischen Zubereitung nach einem der Ansprüche 1 bis 13 zur Behandlung von Nagelfunktionsstörungen oder Nagelerkrankungen.

15. Verwendung einer Zubereitung nach Anspruch 14 gegen Nagelspliss.
